# EUROPEAN PATENT SPECIFICATION

(11) **EP 3 893 675 B1**
(45) Date of publication and mention of the grant of the patent: **15.04.2026**
(21) Application number: 19831581.4
(22) Date of filing: 10.12.2019
(51) Int. Cl.: A23L 33/21, A61K 31/702, A23L 29/30, A61P 1/00, A61P 1/12

(54) **STABLE AQUEOUS COMPOSITION COMPRISING OLIGOSACCHARIDES**
STABILE WÄSSRIGE ZUSAMMENSETZUNG ENTHALTEND OLIGOSACCHARIDE
COMPOSITION AQUEUSE STABLE COMPRENANT DES OLIGOSACCHARIDES

(30) Priority: 14.12.2018 EP 18212616
(43) Date of publication of application: 20.10.2021
(73) Proprietor: Société des Produits Nestlé S.A., 1800 Vevey (CH)
(72) Inventor: MARZORATI, Mattia, 3006 Bern (CH); SPRENGER, Norbert, 1073 SAVIGNY (CH)
(74) Representative: Loiseau, François Michel
(86) International application number: PCT/EP2019/084308
(87) International publication number: WO 2020/120426

(56) References cited:
- EP-A1- 3 375 291
- WO-A1-2018/215960
- WO-A1-2019/012461
- US-A1- 2012 172 307
- US-A1- 2015 183 814
- US-A1- 2016 296 542
- US-A1- 2016 339 046
- WANG J ET AL: "Enzymatic preparation of wheat bran xylooligosaccharides and their stability during pasteurization and autoclave sterilization at low pH", CARBOHYDRATE POLYMERS, APPLIED SCIENCE PUBLISHERS, LTD. BARKING, GB, vol. 77, no. 4, 19 July 2009 (2009-07-19), pages 816 - 821, XP026130502, ISSN: 0144-8617, [retrieved on 20090311], DOI: 10.1016/J.CARBPOL.2009.03.005

## Description

### Field of the invention

The present invention concerns a stable aqueous composition comprising oligosaccharides having a glucose unit at the reducing end and characterised in that the pH of such aqueous composition ranges from 5.5 to 6.5, for example from 5.8 to 6.3. The invention also relate to the use of such aqueous composition as a milk fortifier or supplement for infants, in particular infants who were born by C-section, who were born preterm, or who are small for gestational age and/or who had a low or very low weight at birth (LWB or VLBW). The invention further relates to a method for preventing isomerization of the glucose unit at the reducing end of such oligosaccharides during heat treatment in aqueous compositions.

### Background of the invention

Mother's milk is recommended for all infants. However, in some cases breast feeding is inadequate or unsuccessful for medical reasons or the mother chooses not to breast feed. Infant formulae have been developed for these situations. Fortifiers and supplements have also been developed to enrich mother's milk or infant formula with specific ingredients.

The importance of oligosaccharides which are contained in human breast milk (human milk oligosaccharides, HMOs) is well recognized in the scientific community as being key to support digestive health, gut and/or mucosal maturation, and/or immune maturation in infants. Accordingly, where the feeding of an infant is deprived of such nutrients (as the infant receives infant formulas not containing HMOs) or where the amount of HMOs in the human breast milk or formula the infant receives are not adequate to his needs, a composition providing HMOs in the form of a supplement to be administered to the infant or dissolved into his feeding would be desiderable. Nutritional compositions for infants, which comprise oligosaccharides, are known from e.g., US 2016/296542 A1.

In particular, an aqueous liquid composition comprising HMOs would be needed.

Liquid aqueous compositions for enteral nutrition need to be microbiologically safe.

Often oligosaccharides cannot be concentrated sufficiently in an aqueous solution to reach a microbiologically safe low water activity, because the resulting high density would not allow easy feeding application due to high viscosity and because many oligosaccharides would start to crystallize in a high-density solution.

To address the problem, aseptic filling or sterilization by retorting may be implemented but such procedures provoke during heat treatment isomerization of the reducing terminal monosaccharides of oligosaccharides.

Accordingly, a liquid aqueous composition comprising HMO would be needed that does not incur the isomerization of the reducing terminal monosaccharides of oligosaccharides when subject to heat treatments.

Additionally, storage often alters the oligosaccharide structure and function and consequently the quality of oligosaccharide products in aqueous compositions over time.

Accordingly, a liquid aqueous composition comprising HMO would be needed which shows stability for the oligosaccharides over time.

Furthermore, a liquid aqueous composition comprising HMOs should be characterized by respecting the infant physiology when such composition is administered, either as a stand alone supplement or dissolved in human breast milk.

For example, a too acidic pH of the aqueous composition would not be adapted to administration to infants. This aspect is of particular relevance when the recipient of the aqueous liquid composition is an infant who was born preterm, or who is small for gestational age and/or who had a low or very low weight at birth (LWB or VLBW).

Accordingly, a liquid aqueous composition comprising HMO would be needed which would not alter the infant physiology such as the acid-base balance and to avoid acidosis when such composition is administered.

Accordingly, it is an object of the present invention to identify a solution to prevent isomerization of the reducing terminal monosaccharides of oligosaccharides in aqueous compositions. Additionally, it is a further object of the present invention to prevent degradation of oligosaccharides in aqueous compositions over storage. Ways to prevent the degradation of oligosaccharides in solutions are disclosed in e.g., EP 3 375 291 A1.

Additionally, it is another object of the present invention to provide a liquid aqueous composition comprising HMO which would not alter the infant physiology when such composition is administered.

### Summary of the invention

The inventors surprisingly found that mild acidification at a pH which may range from 5.5 to 6.5, for example from 5.8 to 6.3 or from 5.9 to 6.2, of an aqueous composition comprising at least one oligosaccharide having a glucose unit at the reducing end stabilized such oligosaccharide against isomerization. Very surprisingly, mild acidification also prevented hydrolysis during storage.

In a first aspect, the present invention provides an aqueous composition comprising at least one oligosaccharide having a glucose unit at the reducing end and being characterised in that the pH of such aqueous composition ranges from 5.5 to 6.5, for example from 5.8 to 6.3 or from 5.9 to 6.2, for use in supporting digestive health, gut and/or mucosal maturation, and/or immune maturation in infants, in infants who were born by C-section, or were born preterm, or who are small for gestational age and/or who had a low or very low weight at birth (LWB or VLBW), wherein
the aqueous composition does not comprise other nutrients in addition to the at least one oligosaccharide, and/or
the aqueous composition comprises at least one oligosaccharide having a glucose unit at the reducing end at a concentration ranging from 8 to 35 %w/w of the composition.

In an additional aspect, the present invention provides for a method for preventing isomerization in aqueous compositions during heat treatment of oligosaccharides having a glucose unit at the reducing end such method comprising:
a) Providing an aqueous composition comprising at least one oligosaccharides having a glucose unit at the reducing end;
b) Adjusting the pH of such aqueous composition at a value ranging from 5.5 to 6.5, for example from 5.8 to 6.3 or from 5.9 to 6.2;
c) subjecting the aqueous composition to a heat treatment, wherein

the aqueous composition does not comprise other nutrients in addition to the at least one oligosaccharide, and/or
the aqueous composition comprises at least one oligosaccharide having a glucose unit at the reducing end at a concentration ranging from 8 to 35 %w/w of the composition.

In another aspect, the present invention provides an aqueous composition as defined above, which is packed in a single dose unit.

### Brief description of the drawings

Additional features and advantages of the present invention are described in, and will be apparent from, the description of the embodiments which are set out below with reference to the drawings in which:
**Figure 1** reports the analysis of fructose isomers of 2'FL and LNnT as well as degradation products of 2'FL and LNnT over the sterilization process and storage as described in Example 1.
**Figure 2** reports analysis from Example 2 for 2FL (Fig 2a), LNnT (Fig 2d), the fructose isomers of the added 2'FL (indicated as FL lactulose in the graphs, Fig 2d) and of added LNnT (indicated as LNnT isomer in the graphs, Fig 2e) as well as for their degradation products (lactose, Fig 2c).
**Fig 3** reports pH values over time for solutions of oligosaccharides of Example 2 (respectively starting at pH 6 and 7).

### Detailed description of the invention

The present invention is defined by the claims.

### Definitions

Within the context of the present invention, the term **"monosaccharide"** indicates carbohydrates containing from 3 to 6 carbon atoms. They can be polyhydroxy aldehydes or polyhydroxyketones depending on whether they comprise either an aldehyde or a ketone group, along with -OH substituted carbons in a chain. Polyhydroxy aldehydes are called "aldoses". Polyhydroxyketones are called "Ketoses". Non limiting examples of 6 carbon monosaccharide (hexose) are: allose, altrose, glucose, mannose, gulose, idose, galactose, talose, psicose, fructose, sorbose and tagatose. Non limiting examples of 5 carbon monosaccharide (pentose) are: ribose, arabinose, xylose, lyxose, ribulose and xylulose.

Within the context of the present invention, the term **"oligosaccharide"** indicates a linear or branched saccharide polymer containing a small number (typically two to ten) of simple sugars (5 or 6 membered monosaccharides as above defined).

Within the context of the present invention, the term **"reducing end"** for the oligosaccharide unit identifies the terminal monosaccharide with a free anomeric carbon that is not involved in a glycosidic link.

Within the context of the present invention, the term **"anomeric carbon"** identifies the carbonyl carbon of a monosaccharide in its acyclic form. Depending on the position assumed by -OH group attached to the anomeric carbon when the monosaccharide is in cyclic from (chair conformation), the configuration of such carbon is defined as being α (alpha) or β (beta) if the group -OH is axial or equatorial, respectively.

Within the context of the present invention, the term **"oligosaccharide having a glucose unit at the reducing end"** identifies an oligosaccharide molecule as above defined which presents a glucose unit at the reducing end. Non-limiting examples of such oligosaccharides are:
a) Certain "fucosylated oligosaccharide" that are based on lactose, meaning an oligosaccharide having at least one fucose residue and a glucose at the reducing end. Some examples are 2'-FL (2' fucosyllactose), 3-FL (3-fucosyllactose), difucosyllactose (DFL, also known as LDFT, Lactodifucosyltetraose), lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof.
b) Certain "N-acetylated oligosaccharide(s)" that are based on lactose, meaning an oligosaccharide having at least one N-acetylglucosamine and/or N-acetylgalactosamine residue and a glucose at the reducing end. Some examples are LNT (lacto-N-tetraose), para-lacto-N-neohexaose (para-LNnH), LNnT (lacto-N-neotetraose) or any combination thereof. Other examples are lacto-N-hexaose, lacto-N-neohexaose, para-lacto-N-hexaose, para-lacto-N-neohexaose, lacto-N-octaose, lacto-N- neooctaose, iso- lacto-N-octaose, para- lacto-N-octaose and lacto-N-decaose.
c) Certain "sialylated oligosaccharides" that are based on lactose, meaning an oligosaccharide having at least one sialic acid residue. It has an acidic nature.

Some examples are 3'-SL (3' sialyllactose) and 6'-SL (6' sialyllactose).
In the context of the present invention, within the scope of the expression **"oligosaccharide having a glucose unit at the reducing end"** salts of such oligosaccharides are also comprised. Analogously, when reference is made to specific oligosaccharides deriving from human milk, the salts of such oligosaccharides are also comprised within the scope of such language.

Within the context of the present invention, the expressions "fucosylated oligosaccharides comprising a 2'-fucosyl-epitope" and "2-fucosylated oligosaccharides" encompass fucosylated oligosaccharides with a certain homology of form since they contain a 2'-fucosyl-epitope, therefore a certain homology of function can be expected.

Within the context of the present invention, the expression "at least one fucosylated oligosaccharide" and "at least one N-acetylated oligosaccharide" means "at least one type of fucosylated oligosaccharide" and "at least one type of N-acetylated oligosaccharide".

Within the context of the present invention, the term **"aqueous compositions"** identifies liquid compositions which may be solutions and/or dispersions of the at least one oligosaccharide in an aqueous mean.

Within the context of the present invention, the term **"isomerization"** indicates the process where the tridimensional arrangement of atoms in one molecule changes, producing a new molecule with the same number of atoms but with a different structure.

Within the context of the present invention, the term **"pH modulator"** indicates a substance which is capable of affecting (i.e. decreasing, increasing or stabilizing) the pH of an aqueous solution. Non-limiting examples of pH modulators are strong and mild acids (organic or inorganic), acidic oligosaccharides, strong and mild bases (organic or inorganic) as well as buffers (organic or inorganic). Non limiting examples of organic acids are: Citric acid, Phosphoric acid, Lactic acid and sialic acid. Non limiting examples of inorganic bases are: potassium hydroxide (KOH) and sodium hydroxyde (NaOH). Non limiting examples of acidic oligosaccharides are sialic acid [N-acetyl-neuraminic acid (Neu5Ac )] or uronic acids (glucuronic acid, galacturonic acid).

Within the context of the present invention, the term **"buffer" or "buffering agent"** indicates a substance which is capable of stabilizing the pH of an aqueous solution within a certain narrow pH range. Buffering agents may be combined and/or dissolved in water to provide a buffering solution which are also comprised within the scope of the term "buffer" and/or "buffering agent". Non-limiting examples of buffering agents are: citric acid, acetic acid, phosphate salts (sodium or potassium). Non limiting examples of buffering solutions are: Phosphate buffer (based on 2 phosphates salts, for example sodium phosphate monobasic and sodium phosphate dibasic) and citrate-phosphate buffer (for example Mcllvaine buffer - based on citric acid and disodiumphosphate)
Within the context of the present invention, the term **"fortifier"** refers to a composition which comprises one or more nutrients having a nutritional benefit for infants.

By the term **"milk fortifier",** it is meant any composition used to fortify or supplement either human breast milk, infant formula, growing-up milk or human breast milk fortified with other nutrients. Accordingly, the human milk fortifier of the present invention can be administered after dissolution in human breast milk, infant formula, growing-up milk or human breast milk fortified with other nutrients or otherwise it can be administered as a stand alone composition.

When administered as a stand-alone composition, the human milk fortifier of the present invention can be also identified as being a **"supplement".** In one embodiment, the milk fortifier of the present invention is a supplement.

By the term **"human milk fortifier**", it is meant any composition used to fortify or supplement human breast milk, or human breast milk fortified with other nutrients.

Within the context of the present invention, the expression "composition having a pH ranging from value X to value Y" identifies compositions having a pH range which has one specified value within the indicated range (extremes X and Y of the range being included) as well as compositions having a pH which varies within the indicated range (extremes X and Y of the range being included).

### Embodiments of the invention

The present invention, which is defined by the claims, relates to :
An aqueous composition comprising at least one oligosaccharide having a glucose unit at the reducing end and having pH ranging from 5.5 to 6.5 for use in supporting digestive health, gut and/or mucosal maturation and/or immune maturation in infants who were born by C-section or were born preterm, or who are small for gestational age and/or who had a low or very low weight at birth (LWB or VLBW), wherein
the aqueous composition doesn't comprise other nutrients in addition to the at least one oligosaccharide, and/or
the aqueous composition comprises at least one oligosaccharide having a glucose unit at the reducing end at a concentration ranging from 8 to 35%w/w of the composition.
It also relates to
A method for preventing isomerization in aqueous compositions during heat treatment of oligosaccharides having a glucose unit at the reducing end such method comprising: a) Providing an aqueous composition comprising at least one oligosaccharide having a glucose unit at the reducing end;
b) Adjusting the pH of such aqueous composition at a value ranging from 5.5 to 6.5;
c) subjecting the aqueous composition to a heat treatment wherein
the aqueous composition does not comprise other nutrients in addition to the at least one oligosaccharide, and/or
the aqueous composition comprises at least one oligosaccharide having a glucose unit at the reducing end at a concentration ranging from 8 to 35%w/w of the composition.

### Oligosaccharides

The aqueous composition according to the present invention comprises two or more oligosaccharides having a glucose unit at the reducing end.

In one embodiment, the aqueous composition according to the present invention comprises two oligosaccharides having a glucose unit at the reducing end.

In one embodiment, the aqueous composition according to the present invention comprises three oligosaccharides having a glucose unit at the reducing end.

In one embodiment, the aqueous composition according to the present invention comprises five oligosaccharides having a glucose unit at the reducing end.

In one embodiment, the aqueous composition according to the present invention comprises six oligosaccharides having a glucose unit at the reducing end.

In one embodiment, the aqueous composition according to the present invention comprises seven oligosaccharides having a glucose unit at the reducing end.

In one embodiment, in the aqueous composition according to the present invention, the at least one oligosaccharide having a glucose unit at the reducing end is selected in the group consisting of: 2'-FL, 3'-SL, 6'-SL, DFL, LNnT, LNT and 3-FL.

In another embodiment, in the aqueous composition according to the present invention, the at least one oligosaccharide having a glucose unit at the reducing end is selected in the group consisting of: 2'-FL, 3'-SL, 6'-SL, DFL, LNnT and LNT.

In one embodiment, the aqueous composition according to the present invention comprises two oligosaccharides having a glucose unit at the reducing end and being selected in the group consisting of: 2'-FL, 3-FL, 3'-SL, 6'-SL, DFL, LNnT and LNT, for example being 2'-FL and LNnT.

In one embodiment, the present invention provides an aqueous composition as above defined which comprises 2'-FL and LNnT in a ratio of 10:1.

In another embodiment, the present invention provides an aqueous composition as above defined which comprises 2'-FL and LNnT in a ratio of 2:1.

In one embodiment, the aqueous composition according to the present invention comprises three oligosaccharides having a glucose unit at the reducing end and being being selected in the group consisting of: 2'-FL, 3-FL, 3'-SL, 6'-SL, DFL, LNnT and LNT.

In one embodiment, the present invention provides an aqueous composition as above defined which comprises 2'-FL, DFL and LNT in a ratio of 10:1: 3.33.

In one embodiment, the aqueous composition according to the present invention comprises five oligosaccharides having a glucose unit at the reducing end and being selected in the group consisting of: 2'-FL, 3-FL, 3'-SL, 6'-SL, DFL, LNnT and LNT, for example being 2'-FL, 3'-SL, 6'-SL, DFL, and LNT.

In another embodiment, the present invention provides an aqueous composition as above defined which comprises 2'-FL, DFL, LNT, 6'-SL and 3'-SL in a ratio of 10:1: 3.33: 1.7: 1.2.

In one embodiment, the aqueous composition according to the present invention comprises six oligosaccharides having a glucose unit at the reducing end and being selected in the group consisting of: 2'-FL, 3-FL, 3'-SL, 6'-SL, DFL, LNnT and LNT, for example being 2'-FL, 3'-SL, 6'-SL, DFL, and LNT

In one embodiment, the present invention provides an aqueous composition as above defined which comprises 2'-FL, DFL, LNT, LNnT, 6'-SL and 3'-SL in a ratio of 10:1: 3.33: 1.1: 1.7: 1.2.

In one embodiment, the aqueous composition according to the present invention comprises seven oligosaccharides having a glucose unit at the reducing end and being 2'-FL, 3-FL, 3'-SL, 6'-SL, DFL, LNnT and LNT.

In another embodiment, the present invention provides an aqueous composition as above defined which comprises 2'-FL, DFL, 3-FL, LNT, LNnT, 6'-SL and 3'-SL in a ratio of 10:1: 2.5: 3.33: 1.1: 1.7: 1.2.

### pH range

The aqueous composition according to the invention has a pH ranging from 5.5 to 6.5.

In a further embodiment, the aqueous composition according to the invention has a pH ranging from 5.8 to 6.3.

In another embodiment, the aqueous composition according to the invention has a pH ranging from 5.9 to 6.2.

In another embodiment, the aqueous composition according to the invention has a pH around 6.

### pH modulators

As it can be understood by the skilled person, the pH of the aqueous composition may be also affected by the intrinsic acidity/basicity of the ingredients of the composition and may be then modulated by the use of an appropriate pH modulator.

For example, human milk oligosaccharides which are added in the crystalline form may bring a contribution in the direction of an acidic pH if the material contain residual acetic acid from crystallization process. Such acidic pH may be adjusted and stabilized within the pH range according to the invention via the use of appropriate pH modulators.

In one embodiment, the aqueous composition according to the invention has a pH ranging from 5.5 to 6.5, for example from 5.8 to 6.3 or from 5.9 to 6.2, which is achieved via the addition of one or more pH modulators.

In one embodiment, the pH modulator is an organic acid. Any organic acid may be used as technical aid to generate stable oligosaccharide solutions. Preferably, citric acid, sialic acid [also known as N-acetyl-neuraminic acid (Neu5Ac)], malic acid, lactic acid maybe used.

In another embodiment, the pH modulator is an acidic oligosaccharide. In this case, sialic acid (N-acetyl-neuraminic acid,Neu5Ac ) containing oligosaccharides or uronic acids (glucuronic acid, galacturonic acid) containing oligosaccharides may be used.

Typical examples would pectin derived oligosaccharides for uronic acid containing oligosaccharides.

Advantageously, if sialic acid [N-acetyl-neuraminic acid (Neu5Ac )] containing oligosaccharides are part of the oligosaccharide mixture in the compositions according to the invention, the acidic oligosaccharide per se may be used to acidify and stabilize the solution so that the acidic oligosaccharide plays also the role of a pH modulator. Typical examples would be 3' and 6'-sialyllactose for sialic acid containing oligosaccharides.

In another embodiment, the pH modulator is an inorganic base, for example KOH or NaOH.

In one embodiment, the pH modulator also consists of or comprises a buffering agent, for example phosphate buffer, or citrate-phosphate buffer (also known as Mcllvaine buffer, based on a mixture of citric acid and disodiumphosphate).

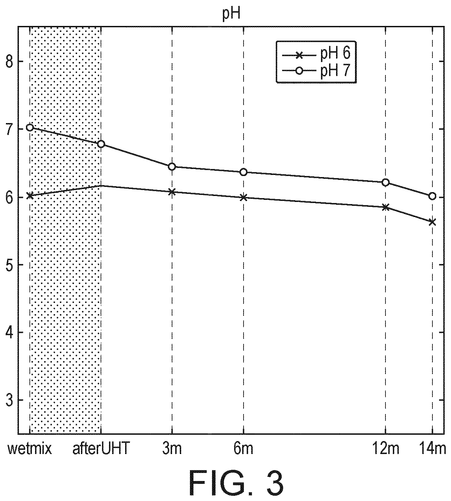

As it can be inferred from the data reported in Fig 3, Example 2, there is surprisingly a natural decrease in the pH levels of the aqueous compositions of the invention. As it is understood that the pH should remain relatively stable within certain values over storage, in order to guarantee that the aqueous composition comprising HMOs remains adapted to administration to infants , particularly preterm infants, and respecting their physiology.

Accordingly, the option of including a buffering agent in the composition offers the additional advantage of allowing the pH to remain stable over time.

### Aqueous compositions

In one embodiment, the present invention provides an aqueous composition comprising at least one oligosaccharide having a glucose unit at the reducing end at a concentration ranging from 8 to 35%w/w of the composition, wherein the pH of such aqueous composition ranges from 5.5 to 6.5, for example from 5.8 to 6.3 or from 5.9 to 6.2, and the composition doesn't comprise other nutrients in addition to the at least one oligosaccharide.

In one embodiment, the present invention provides an aqueous composition comprising at least one oligosaccharides having a glucose unit at the reducing end at a concentration ranging from 8 to 35%w/w of the composition, a pH modulator and wherein the pH of such aqueous composition ranges from 5.5 to 6.5, for example from 5.8 to 6.3 or from 5.9 to 6.2, and the composition doesn't comprise other nutrients in addition to the oligosaccharide.

In one embodiment, the present invention provides an aqueous composition comprising at least one oligosaccharides having a glucose unit at the reducing end at a concentration ranging from 8 to 35%w/w of the composition, a pH modulator, a buffering agent and wherein the pH of such aqueous composition ranges from 5.5 to 6.5, for example from 5.8 to 6.3 or from 5.9 to 6.2, and the composition doesn't comprise other nutrients in addition to the oligosaccharide.

In another embodiment, the present invention provides an aqueous composition comprising two oligosaccharides having a glucose unit at the reducing end at a concentration ranging from 8 to 35%w/w of the composition, wherein the pH of such aqueous composition ranges from 5.5 to 6.5, for example from 5.8 to 6.3 or from 5.9 to 6.2, and the composition doesn't comprise other nutrients in addition to the oligosaccharidse.

In another embodiment, the present invention provides an aqueous composition comprising two oligosaccharides having a glucose unit at the reducing end at a concentration ranging from 8 to 35%w/w of the composition, a pH modulator and wherein the pH of such aqueous composition ranges from 5.5 to 6.5, for example from 5.8 to 6.3 or from 5.9 to 6.2, and the composition doesn't comprise other nutrients in addition to the oligosaccharides.

In another embodiment, the present invention provides an aqueous composition comprising two oligosaccharides having a glucose unit at the reducing end at a concentration ranging from 8 to 35%w/w of the composition, a pH modulator, a buffering agent and wherein the pH of such aqueous composition ranges from 5.5 to 6.5, for example from 5.8 to 6.3 or from 5.9 to 6.2, and the composition doesn't comprise other nutrients in addition to the oligosaccharides.

In another embodiment, the present invention provides an aqueous composition comprising two oligosaccharides having a glucose unit at the reducing end at a concentration ranging from 8 to 35%w/w of the composition, a pH modulator, a buffering agent and wherein the pH of such aqueous composition ranges from 5.5 to 6.5, for example from 5.8 to 6.3 or from 5.9 to 6.2, the oligosaccharides are 2'-FL and LNnT and the composition doesn't comprise other nutrients in addition to the oligosaccharides.

In a further embodiment, the present invention provides an aqueous composition comprising five oligosaccharides having a glucose unit at the reducing end at a concentration ranging from 8 to 35%w/w of the composition, wherein the pH of such aqueous composition ranges from 5.5 to 6.5, for example from 5.8 to 6.3 or from 5.9 to 6.2, and the composition doesn't comprise other nutrients in addition to the oligosaccharidse.

In a further embodiment, the present invention provides an aqueous composition comprising five oligosaccharides having a glucose unit at the reducing end at a concentration ranging from 8 to 35%w/w of the composition, a pH modulator and wherein the pH of such aqueous composition ranges from 5.5 to 6.5, for example from 5.8 to 6.3 or from 5.9 to 6.2, and the composition doesn't comprise other nutrients in addition to the oligosaccharides.

In a further embodiment, the present invention provides an aqueous composition comprising five oligosaccharides having a glucose unit at the reducing end at a concentration ranging from 8 to 35%w/w of the composition, a pH modulator, a buffering agent and wherein the pH of such aqueous composition ranges from 5.5 to 6.5, for example from 5.8 to 6.3 or from 5.9 to 6.2, and the composition doesn't comprise other nutrients in addition to the oligosaccharides.

In a further embodiment, the present invention provides an aqueous composition comprising five oligosaccharides having a glucose unit at the reducing end at a concentration ranging from 8 to 35%w/w of the composition, a pH modulator, a buffering agent and wherein the pH of such aqueous composition ranges from 5.5 to 6.5, for example from 5.8 to 6.3 or from 5.9 to 6.2, the oligosaccharides are 2'-FL, 3'-SL, 6'-SL, DFL and LNT and the composition doesn't comprise other nutrients in addition to the oligosaccharides.

In a still further embodiment, the present invention provides an aqueous composition comprising six oligosaccharides having a glucose unit at the reducing end at a concentration ranging from 8 to 35%w/w of the composition, wherein the pH of such aqueous composition ranges from 5.5 to 6.5, for example from 5.8 to 6.3 or from 5.9 to 6.2, and the composition doesn't comprise other nutrients in addition to the oligosaccharide.

In a still further embodiment, the present invention provides an aqueous composition comprising six oligosaccharides having a glucose unit at the reducing end at a concentration ranging from 8 to 35%w/w of the composition, a pH modulator and wherein the pH of such aqueous composition ranges from 5.5 to 6.5, for example from 5.8 to 6.3 or from 5.9 to 6.2, and the composition doesn't comprise other nutrients in addition to the oligosaccharides.

In a still further embodiment, the present invention provides an aqueous composition comprising six oligosaccharides having a glucose unit at the reducing end at a concentration ranging from 8 to 35%w/w of the composition, a pH modulator, a buffering agent and wherein the pH of such aqueous composition ranges from 5.5 to 6.5, for example from 5.8 to 6.3 or from 5.9 to 6.2, and the composition doesn't comprise other nutrients in addition to the oligosaccharides.

In a still further embodiment, the present invention provides an aqueous composition comprising six oligosaccharides having a glucose unit at the reducing end at a concentration ranging from 8 to 35%w/w of the composition, a pH modulator, a buffering agent and wherein the pH of such aqueous composition ranges from 5.5 to 6.5, for example from 5.8 to 6.3 or from 5.9 to 6.2, the oligosaccharides are 2'-FL, 3'-SL, 6'-SL, DFL, LNnT and LNT and the composition doesn't comprise other nutrients in addition to the oligosaccharides.

### Format

By having a liquid form, aqueous compositions according to the present invention present some particular benefits. For example, they may be more conveniently packed to deliver calibrated drops of a certain weight or volume.

In some embodiment, aqueous compositions of the present invention may be packed in single doses in such a way that calibrated drops of a certain weight or volume are delivered while avoiding contamination of the remaining liquid due to manipulation and subsequent uses.

In one embodiment, the liquid aqueous composition according to the present invention is presented in single dose units which are packed in plastic material. In one embodiment, such plastic material is flexible and squeezable. In one embodiment, such plastic material may be polypropylene (PP) or Polyethylene (PE). In one embodiment, polypropylene may be low density (LD PE) or high density (HD PE).In addition, aqueous compositions are easy to mix with compositions to be fortified, whereas the powder ones can, in some cases, form lumps.

### Supplements

In one embodiment, the aqueous composition according to the present invention and above described is a supplement. In such embodiment, the aqueous composition of the invention is administered as a stand alone composition. In such embodiment, the aqueous composition of the invention is administered as a stand alone composition and is packed in single doses.

### Fortifiers

In one embodiment, the aqueous composition according to the present invention is a milk fortifier. In such embodiment, the aqueous composition of the invention may be packed in single doses.

### Experimental Section

### Method for quantitative determination of 2'FL and LNnT (Austin, etal, Molecules, 23 (2018), 2650)

An oligosaccharide (laminaritriose) internal standard is mixed with the oligosaccharide solution and the oligosaccharides are fluorescently labelled by reaction of the 2-anthranilic acid amide (2AB) with the OS reducing end via formation of a Schiff base. The double bond is then reduced by reaction with sodium cyanoborohydride to give a stable OS-2AB derivative.

Labelled samples are diluted with acetonitrile (ACN) prior to injection on an ultra high performance liquid chromatography (UHPLC) system equipped with hydrophilic interaction liquid chromatography (HILIC) trapping and analytical columns. Labelled OS are detected by a fluorimeter.

Oligosaccharide concentrations are determined from a standard curve using the relative response of the oligosaccharide (OS) to the internal standard (Laminaritriose).

### Example 1

Oligosaccharides 2'Fucosyllactose (2'FL) and Lacto-N-neotetraose (LNnT) were mixed in a 2:1 ratio (w/w) and dissolved in water to a final concentration of 10% (w/v). Solutions were acidified to pH 6 with **citric acid** and subjected to heat treatment (UHT) followed by aseptic filling of small monodose bottles. Solutions were analysed before and after filling of bottles as well as after different times of storage at 37°C (accelerated storage condition).

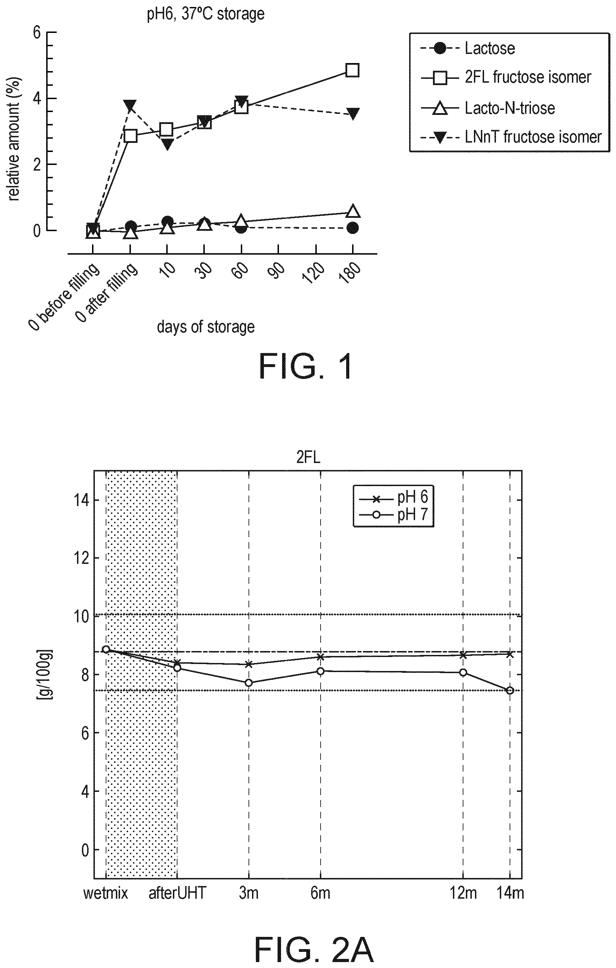

Analysis was done by HPLC using a TSK gel Amide-80 (150 mm x 4.6 mm, particle size: 3µm) column and detection via a charged aerosol detector. For the fructose isomers of the added 2'FL and LNnT as well as for their degradation products (fucose, lactose and Lacto-N-triose) quantification was done using the relative response of the oligosaccharide to a standard curve. Fucose, Lactose, 2FL, LNnT, 2'-fucosyl-lactulose and LNnT fructose isomer were used as standards. Lacto-N-triose contents were calculated with the calibration curve of LNnT. Results are reported in Fig 1. All data are expressed as % of added oligosaccharides.

The results indicate that at pH 6, very limited formation of such degradation and/or isomerization products is observed, either at aseptic filling or during storage under accelerated conditions, thus showing the stability of the aqueous solution of the invention under the tested conditions.

### Example 2

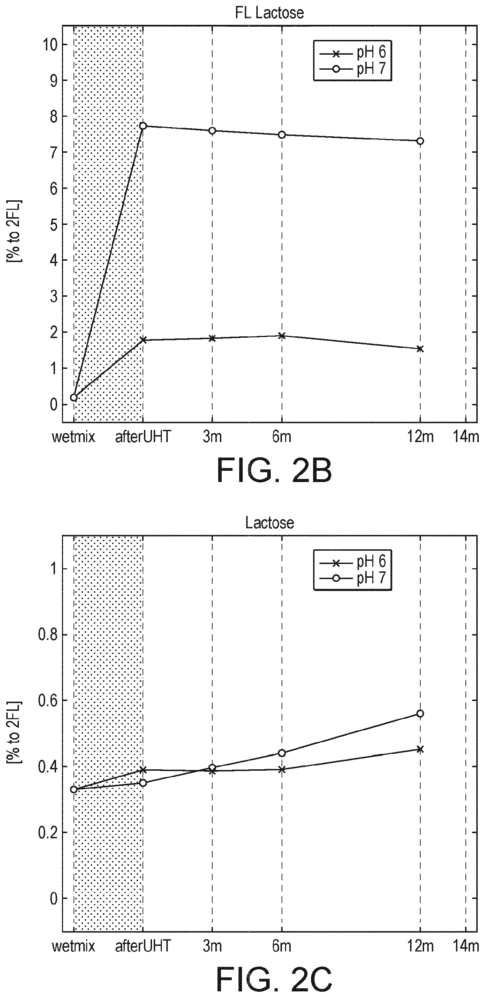

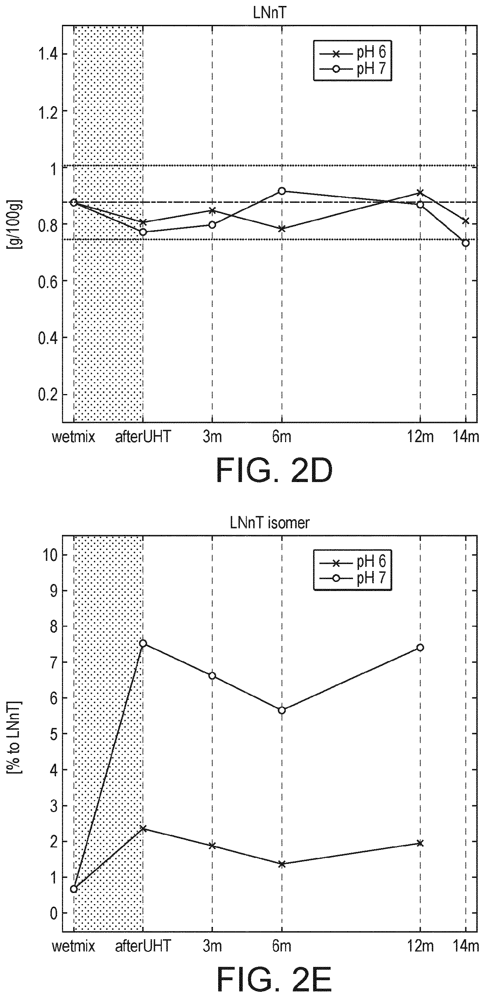

Oligosaccharides 2'Fucosyllactose (2'FL) and Lacto-N-neotetraose (LNnT) were mixed in a 10:1 ratio (w/w) and dissolved in water to a final concentration of 10% (w/v). Out of this solution, three samples were generated respectively having pH 6 and7 (by addition of KOH) Such samples were subjected to heat treatment (UHT) followed by aseptic filling of small monodose bottles. Solutions at different pHs were analysed before and after filling of bottles as well as after different times of storage at room temperature (21-26 °C). Analysis was done by HPLC using a TSK gel Amide-80 (150 mm x 4.6 mm, particle size: 3µm) column and detection via a charged aerosol detector. F or 2'FL (Fig 2a), LNnT (Fig 2d), the fructose isomers of the added 2'FL (indicated as FL lactulose in the graphs, Fig 2d) and of added LNnT (indicated as LNnT isomer in the graphs, Fig 2e) as well as for their degradation products (lactose, Fig 2c). The pH of the solution was also measured. All data are expressed as % of added oligosaccharides.

The results indicate that at pH 7 2FL undergoes significant transformation as it appears from the decreased levels at asepting filling and over time Fig2a). This finding is also confirmed by considerable FL lactulose at this pH (Fig 2b and Fig 2e, respectively).

At pH 6, the situation is considerably different. Firstly, 2FL and LNnT amounts appear to be much more stable (Fig 2a and Fig2d, respectively). Additionally, FL lactulose as well as LNnT isomers formation is much lower (Fig2b and Fig2e respectively) indicating stability of the oligosaccharides in the aqueous solution against isomerization. Lastly, lactose levels remain stable over time also indicating stability against hydrolysis of the oligosaccharides.

### Example 3

Oligosaccharides 2'Fucosyllactose (2'FL) and Lacto-N-neotetraose (LNnT) were mixed in a 10:1 ratio (w/w) and dissolved in buffered water (phosphate buffer as before defined) to a final concentration of 10% (w/v). Solution is submitted to a sterile filtration and filled in plastic monodose unit. Resulting pH of the solution before and after sterile filtration was around 6.

### Example 4

0.91 of LNnT and 9.09 g of 2'-FL have been dissolved in water (to a final volume of 100 ml), to a final concentration of 10 g/l (10% w/v).

From the obtained solution 2 aliquots have been obtained (HMO's solutions). In one aliquot the pH has been adjust to 5 using a citric acid solution (10% w/w). In another aliquot pH has been adjusted to 6 using KOH solution (10% w/w).

4 g of the above mentioned HMO's solutions have been added to 2 samples of human breast milk (20 g each). The initial pH of the pure breastmilk has been measured and recorded as being 7.3.

The final pH of the samples obtained by dissolution of the HMO's solutions in human breast milk has been measured, data are shown in the below table 1.

**Table 1**

| | **pH (@ 20 °C)** |
|---|---|
| Human Breastmilk | 7.30 |
| 20 g Human Breast milk + 4 g HMO solution @ pH 4.99 | 6.94 |
| 20 g Human Breast milk + 4 g HMO solution @ pH 6.00 | 7.18 |

## Claims

1. Aqueous composition comprising at least one oligosaccharide having a glucose unit at the reducing end and having pH ranging from 5.5 to 6.5 for use in supporting digestive health, gut and/or mucosal maturation and/or immune maturation in infants who were born by C-section or were born preterm, or who are small for gestational age and/or who had a low or very low weight at birth (LWB or VLBW), wherein
the aqueous composition does not comprise other nutrients in addition to the at least one oligosaccharide, and/or
the aqueous composition comprises at least one oligosaccharide having a glucose unit at the reducing end at a concentration ranging from 8 to 35%w/w of the composition.

2. Aqueous composition for use according to claim 1 which is a milk fortifier or a supplement.

3. Aqueous composition for use according to claim 1 or 2 wherein the pH ranges from 5.8 to 6.3, for example from 5.9 to 6.1.

4. Aqueous composition for use according to any of claims 1 to 3 which comprises a pH modulator.

5. Aqueous composition for use according to any of claims 1 to 4 which further comprises a buffering agent.

6. Aqueous composition for use according to any of claims 1 to 5 wherein the at least one oligosaccharide is selected from the group consisting of: 2'-FL, 3'-SL, 6'-SL, DFL, LNnT, LNT and 3-FL.

7. Aqueous composition for use according to any of claims 1 to 5 which comprises two oligosaccharides having a glucose unit at the reducing end which are selected in the group consisting of: 2'-FL, 3-FL, 3'-SL, 6'-SL, DFL, LNnT and LNT, for example being 2'-FL and LNnT.

8. Aqueous composition for use according to any of claims 1 to 5 which comprises five oligosaccharides having a glucose unit at the reducing end which are selected in the group consisting of: 2'-FL, 3-FL, 3'-SL, 6'-SL, DFL, LNnT and LNT, for example being 2'-FL, 3'-SL, 6'-SL, DFL, and LNT.

9. Aqueous composition for use according to any of claims 1 to 8 which is packed in a single dose unit.

10. Method for preventing isomerization in aqueous compositions during heat treatment of oligosaccharides having a glucose unit at the reducing end such method comprising:
a) Providing an aqueous composition comprising at least one oligosaccharide having a glucose unit at the reducing end;
b) Adjusting the pH of such aqueous composition at a value ranging from 5.5 to 6.5;
c) subjecting the aqueous composition to a heat treatment
wherein
the aqueous composition does not comprise other nutrients in addition to the at least one oligosaccharide, and/or
the aqueous composition comprises at least one oligosaccharide having a glucose unit at the reducing end at a concentration ranging from 8 to 35%w/w of the composition.

## Patentansprüche

1. Wässrige Zusammensetzung, umfassend mindestens ein Oligosaccharid, das eine Glucoseeinheit an dem reduzierenden Ende aufweist und einen pH-Wert in einem Bereich von 5,5 bis 6,5 aufweist, zur Verwendung bei einem Unterstützen der Verdauungsgesundheit, der Darm- und/oder Schleimhautreifung und/oder der Immunreifung bei Säuglingen, die per Kaiserschnitt geboren wurden oder vorzeitig geboren wurden, oder die klein für das Gestationsalter sind und/oder die ein niedriges oder sehr niedriges Geburtsgewicht (LWB oder VLBW) hatten, wobei
die wässrige Zusammensetzung keine anderen Nährstoffe zusätzlich zu dem mindestens einen Oligosaccharid umfasst, und/oder
die wässrige Zusammensetzung mindestens ein Oligosaccharid umfasst, das eine Glucoseeinheit an dem reduzierenden Ende in einer Konzentration in einem Bereich von 8 bis 35 Gew.-% der Zusammensetzung aufweist.

2. Wässrige Zusammensetzung zur Verwendung nach Anspruch 1, die ein Milchanreicherungsmittel oder ein Nahrungsergänzungsmittel ist.

3. Wässrige Zusammensetzung zur Verwendung nach Anspruch 1 oder 2, wobei der pH-Wert in einem Bereich von 5,8 bis 6,3, zum Beispiel von 5,9 bis 6,1 liegt.

4. Wässrige Zusammensetzung zur Verwendung nach einem der Ansprüche 1 bis 3, die einen pH-Modulator umfasst.

5. Wässrige Zusammensetzung zur Verwendung nach einem der Ansprüche 1 bis 4, die ferner ein Puffermittel umfasst.

6. Wässrige Zusammensetzung zur Verwendung nach einem der Ansprüche 1 bis 5, wobei das mindestens eine Oligosaccharid aus der Gruppe ausgewählt ist, bestehend aus: 2'-FL, 3'-SL, 6'-SL, DFL, LNnT, LNT und 3-FL.

7. Wässrige Zusammensetzung zur Verwendung nach einem der Ansprüche 1 bis 5, die zwei Oligosaccharide umfasst, die eine Glucoseeinheit an dem reduzierenden Ende aufweisen, die aus der Gruppe ausgewählt sind, bestehend aus: 2'-FL, 3-FL, 3'-SL, 6'-SL, DFL, LNnT und LNT, zum Beispiel 2'-FL und LNnT.

8. Wässrige Zusammensetzung zur Verwendung nach einem der Ansprüche 1 bis 5, die fünf Oligosaccharide umfasst, die eine Glucoseeinheit an dem reduzierenden Ende aufweisen, die aus der Gruppe ausgewählt sind, bestehend aus: 2'-FL, 3-FL, 3'-SL, 6'-SL, DFL, LNnT und LNT, zum Beispiel 2'-FL, 3'-SL, 6'-SL, DFL und LNT.

9. Wässrige Zusammensetzung zur Verwendung nach einem der Ansprüche 1 bis 8, die in einer Einzeldosiseinheit verpackt ist.

10. Verfahren zum Verhindern einer Isomerisierung in wässrigen Zusammensetzungen während einer Wärmebehandlung von Oligosacchariden, die eine Glucoseeinheit an dem reduzierenden Ende aufweisen, ein derartiges Verfahren umfassend:
a) Bereitstellen einer wässrigen Zusammensetzung, umfassend mindestens ein Oligosaccharid, das eine Glucoseeinheit an dem reduzierenden Ende aufweist;
b) Einstellen des pH-Werts einer derartigen wässrigen Zusammensetzung auf einen Wert in einem Bereich von 5,5 bis 6,5;
c) Unterziehen der wässrigen Zusammensetzung einer Wärmebehandlung
wobei
die wässrige Zusammensetzung keine anderen Nährstoffe zusätzlich zu dem mindestens einen Oligosaccharid umfasst, und/oder
die wässrige Zusammensetzung mindestens ein Oligosaccharid umfasst, das eine Glucoseeinheit an dem reduzierenden Ende in einer Konzentration in einem Bereich von 8 bis 35 Gew.-% der Zusammensetzung aufweist.

## Revendications

1. Composition aqueuse comprenant au moins un oligosaccharide ayant une unité de glucose à l'extrémité réductrice et ayant un pH compris entre 5,5 et 6,5, pour utilisation dans le fait de maintenir une santé digestive, la maturation intestinale et/ou muqueuse et/ou la maturation immunitaire chez les nourrissons nés par césarienne ou nés prématurément, ou qui sont petits pour l'âge gestationnel et/ou qui avaient un poids faible ou très faible à la naissance (LWB ou VLBW), dans laquelle
la composition aqueuse ne comprend pas d'autres nutriments en plus de l'au moins un oligosaccharide, et/ou
la composition aqueuse comprend au moins un oligosaccharide ayant une unité de glucose à l'extrémité réductrice à une concentration allant de 8 à 35 % p/p de la composition.

2. Composition aqueuse pour utilisation selon la revendication 1, qui est un fortifiant pour le lait ou un complément.

3. Composition aqueuse pour utilisation selon la revendication 1 ou 2, dans laquelle le pH est compris entre 5,8 et 6,3, par exemple entre 5,9 et 6,1.

4. Composition aqueuse pour utilisation selon l'une quelconque des revendications 1 à 3, qui comprend un modulateur de pH.

5. Composition aqueuse pour utilisation selon l'une quelconque des revendications 1 à 4, qui comprend en outre un agent tampon.

6. Composition aqueuse pour utilisation selon l'une quelconque des revendications 1 à 5, dans laquelle l'au moins un oligosaccharide est choisi dans le groupe constitué de : 2'-FL, 3'-SL, 6'-SL, DFL, LNnT, LNT et 3-FL.

7. Composition aqueuse pour utilisation selon l'une quelconque des revendications 1 à 5, qui comprend deux oligosaccharides ayant une unité de glucose à l'extrémité réductrice, qui sont choisis dans le groupe constitué de : 2'-FL, 3-FL, 3'-SL, 6'-SL, DFL, LNnT et LNT, par exemple 2'-FL et LNnT.

8. Composition aqueuse pour utilisation selon l'une quelconque des revendications 1 à 5, qui comprend cinq oligosaccharides ayant une unité de glucose à l'extrémité réductrice, qui sont choisis dans le groupe constitué de : 2'-FL, 3-FL, 3'-SL, 6'-SL, DFL, LNnT et LNT, par exemple 2'-FL, 3'-SL, 6'-SL, DFL et LNT.

9. Composition aqueuse pour utilisation selon l'une quelconque des revendications 1 à 8, qui est conditionnée dans une unité de dose unique.

10. Procédé destiné à empêcher l'isomérisation dans des compositions aqueuses pendant le traitement thermique d'oligosaccharides ayant une unité de glucose à l'extrémité réductrice, le tel procédé comprenant :
a) La fourniture d'une composition aqueuse comprenant au moins un oligosaccharide ayant une unité de glucose à l'extrémité réductrice ;
b) L'ajustement du pH de telle composition aqueuse à une valeur comprise entre 5,5 et 6,5 ;
c) La soumission de la composition aqueuse à un traitement thermique
dans lequel
la composition aqueuse ne comprend pas d'autres nutriments en plus de l'au moins un oligosaccharide, et/ou
la composition aqueuse comprend au moins un oligosaccharide ayant une unité de glucose à l'extrémité réductrice à une concentration allant de 8 à 35 % p/p de la composition.
